# DEMANDE DE BREVET EUROPEEN

(11) **EP 1 586 632 A1**
(43) Date de publication de la demande: **19.10.2005**
(21) Numéro de dépôt: 04405225.6
(22) Date de dépôt: 14.04.2004
(51) Int. Cl.: C12M 1/107, C12M 1/113

(54) **Digesteur-anaérobique**

(71) Demandeur: Hatoum Socrate, 75008 Paris (FR)
(72) Inventeur: Hatoum Socrate, 75008 Paris (FR)
(74) Mandataire: AMMANN PATENTANWÄLTE AG BERN

(57) **Abrégé**

Un digesteur-anaérobique (1) comprend au moins une cuve de digestion (3) pour digérer des déchets surtout émanant d'une entreprise agricole en produisant du biogaz. Le digesteur-anaérobique (1) comprend une ou plusieurs mesures suivantes:
- les cuves sont couvertes d'une isolation (29) éspacée d'une petite distance de la paroi (37) des cuves pour avoir une lame d'air (39) entre l'isolation et la paroi;
- une cuve de préparation (46) comprend un système de mesure à ultrasons pour mesurer la concentration de la matière solide dans la cuve de préparation et est montée sur des capteurs de pesage (47).
- les déchets des plantes sont diminués avant de les alimenter à la cuve de préparation;
- les cuves de digestion (3) sont arrangées chacune dans une cuve extérieure (64) remplie de l'eau chaude afin de maintenir les cuves de digestion (3) à une température optimale pour le processus;
- un co-générateur (19) sert à produire à la fois de l'électricité et de la chaleur pour chauffer les cuves de digestion (3) .

## Description

La présente invention a trait à un digesteur-anaérobique, aussi dénommé méthaniseur, destiné a l'agriculture, selon le préambule de la revendication 1.

Suite à la crise énergétique de 1983, beaucoup de digesteurs anaérobiques ont été construits en Europe et dans le monde, souvent dans les exploitations agricoles. Leur production de biogaz était très basse et ne pouvait pas justifier le travail et les coûts consacrés à leur mise en oeuvre et à leur entretien.

En outre, beaucoup d'éléments et d'équipements nécessaires à leur bon fonctionnement et à leur facilité d'opération manquaient.

Depuis quelques années, en Allemagne, en Autriche et en Suisse, des actions politiques encourageant la méthanisation ont donné une nouvelle impulsion à la construction et à la commercialisation d'une deuxième génération de méthaniseurs compacts, destinés aux petites et moyennes exploitations agricoles. L'introduction de la co-digestion (mélange de différents déchets ) et de la co-génération d'électricité et de chaleur, ont légèrement amélioré la productivité et la rentabilité des installations de méthanisation.

Mais la période de retour sur investissement reste supérieure à 8 ans.

Un objet de l'invention est donc de proposer un méthaniseur dont la mise en oeuvre et l'entretien sont facilités.

Un tel méthaniseur est défini dans la revendication 1. Les revendications suivantes portent sur des exécutions préférées d'un tel méthaniseur.

L'invention sera décrite ci-après à l'appui d'un exemple préféré en se référant aux figures suivantes:

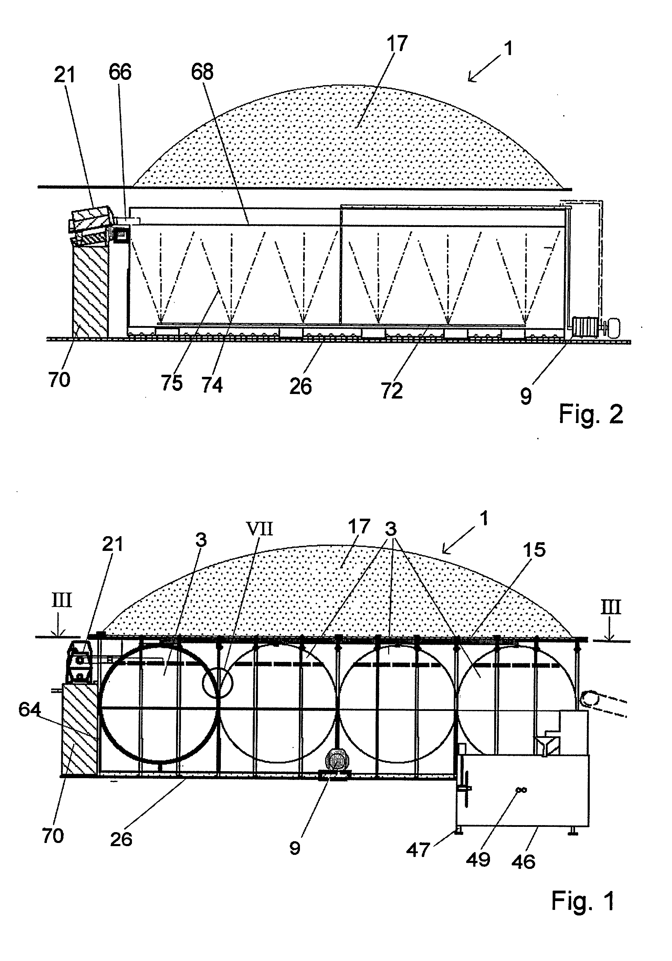

Fig. 1 Elévation schématique d'une installation avec le méthaniseur;
Fig. 2 Vue latérale schématique de l'installation selon la Fig. 1;

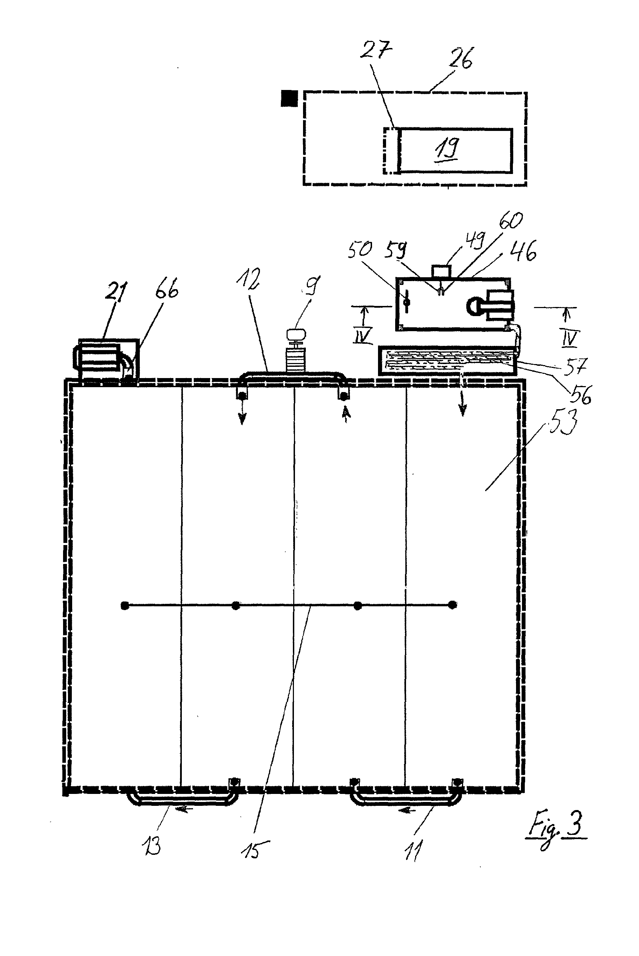

Fig. 3 Vue de dessus schématique selon la ligne III-III de la Fig. 1;

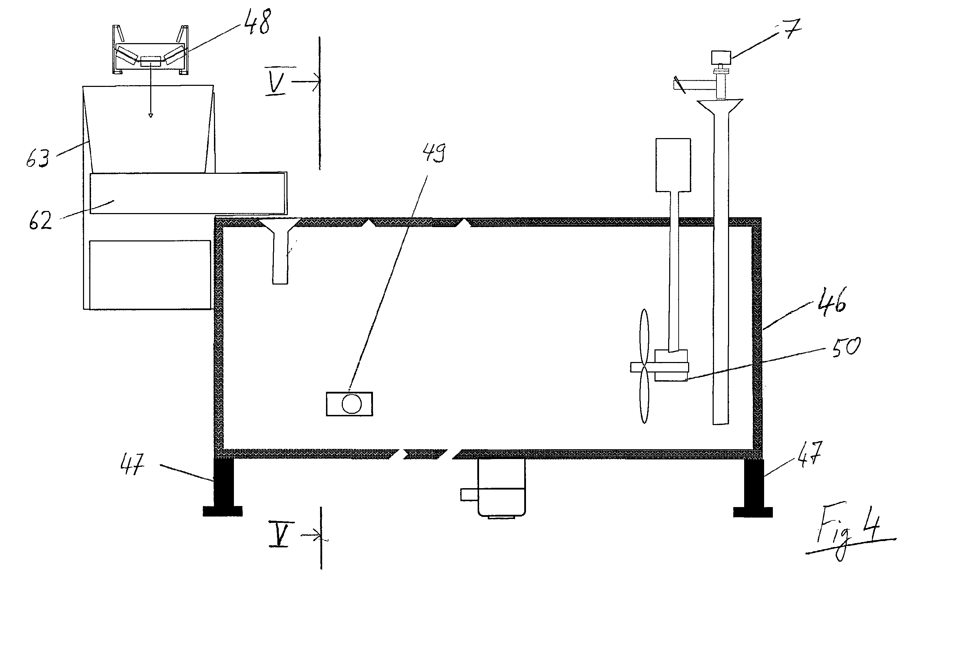

Fig. 4 Coupe longitudinale schématique par la cuve de préparation selon IV-IV in Fig. 3;

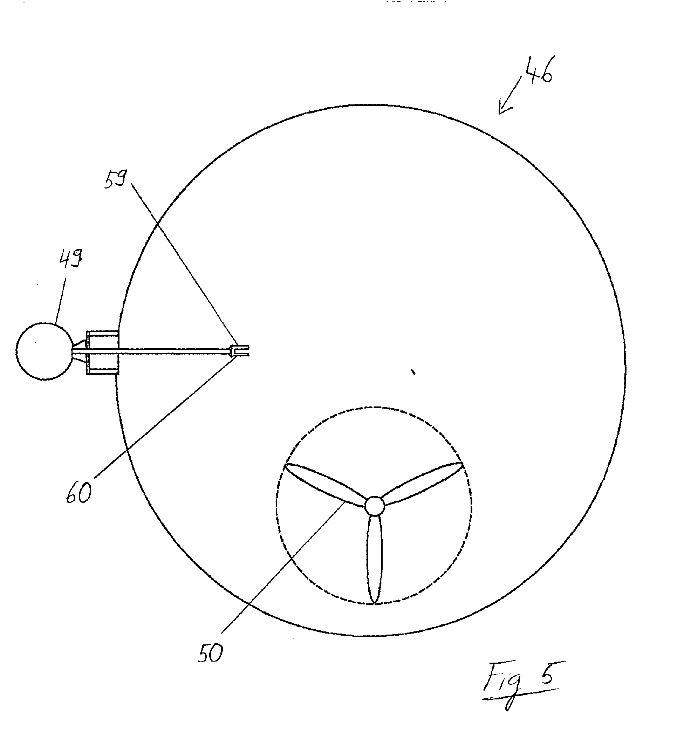

Fig. 5 Coupe transversale schématique par la cuve de préparation selon V-V in Fig. 4;
Fig. 6 Schéma de fonctionnement du détecteur ultrasonique;
Fig. 7 Détail selon cercle VII dans la Figure 1; et
Fig. 8, 9 Construction de l'isolation des cuves en coupes transversale et longitudinale.

Le méthaniseur 1 selon l'invention, aussi dénommé digesteur anaérobique, sert à la digestion anaérobique. Il est constitué d'une ou plusieurs cuves de digestion 3 dans lesquelles sont introduits des déchets encombrants, malodorants et polluants pour y être valorisés et assainis.

Les cuves de digestions 3 sont chauffées, isolées et closes hermétiquement à l'air, l'eau et à la vapeur d'eau.

Le méthaniseur 1 est doté d'appareils de traitement des déchets: de pompes , d'un agitateur 9, de tuyaux d'eau 11, 12, 13 et de gaz 15, d'un conteneur de biogaz 17, d'un co-générateur 19. De plus, il est pourvu de connexions électriques, de relais, d'un système informatique de coordination, de contrôle et de gestion et servant à l'automatisation de l'installation. Ces derniers moyens ne sont pas représentés et peuvent être réalisés selon l'art connu, cet qui permet de s'abstenir d'en faire une description.

La digestion anaérobique (D.A.) est un procédé de transformation en biogaz, en compost (solide) et en effluent (liquide). Ce sont les sous-produits de la digestion des matières organique par des bactéries méthanogènes anaérobiques. Les matières organiques sont contenues dans le digestat introduit dans le méthaniseur. La quantité et la qualité de ces sous-produits dépendent du choix de la température de la digestion et de la durée de séjour ou temps de rétention dans le méthaniseur. Ce temps est déterminé par le volume hydraulique, c.-à.-d. la dimension utile de la ou des cuves de rétention, du choix de leur système de chauffage, d'isolation, et finalement des conditions de rétention: le digestat doit être remué afin d'empêcher le croûtage de sa surface, la température doit être maintenue constante, la composition, la consistance et la température du digestat introduit dans les cuves 3 doivent être contrôlées pour qu'elles soient égales à celles du matériau dans les cuves 3.

Comme digestats peuvent servir les fèces, bouses, urines, crottes et crottins des animaux de la ferme, les restes de plantes cultivées ou sauvages, les déchets des fruits, des racines, des plantes, les balles de céréales et le reste de leur farines et les restes de cantines et de cuisines.

Comme produit du méthaniseur, on obtient du biogaz, du compost (matière solide) et l'effluent.

Le biogaz est produit par la décomposition des matières biodégradables en absence d'oxygène. Le biogaz est constitué à 98% de méthane (CH₄) et de dioxyde de carbone (CO₂).

Le compost est constitué de matières solides non digérées, tels que la lignine et les composés ligno-cellulosiques, et le reste des digestats partiellement ou non digérés, d'une taille plus grande que les mailles du tamis 21 situé à la sortie du digesteur anaérobique. Le compost est désodorisé et peut être distribué sur des gazons, des pelouses, des terrains de golf , de sport etc. Le compost anaérobique peut remplacer les autres composts ainsi que les engrais chimiques.

Les effluents sont les liquides sortant d'un digesteur anaérobique. Ils contiennent des sels minéraux dissous et des particules plus petites que les mailles du tamis 21 à la sortie du méthaniseur 1. Ils sont désodorisés et peuvent aussi être distribués sur les champs à la place de lisiers et du fumier.

Comme déjà mentionné, le digestat est soumis à une contrôle rigide pour qu'il entre dans les cuves en condition optimale. A cette fin, le digestat est préparé dans la cuve de préparation 23.

Dans le cadre de la présente description, par matières sèches (MS) on comprend les constituants non liquides de tout produit biologique; et par matières sèches organiques (MSO), les constituants non minéraux et biodégradables des matières sèches. Ce sont surtout eux qui sont méthanisées et qui, par conséquent, perdent une partie de leur poids.

Pour effectuer la méthanisation, se sont avérés importants les paramètres suivants:

### Temps de rétention

Le temps de rétention représente le temps durant lequel le digestat est digéré dans le digesteur. A priori, plus ce temps augmente, plus le digestat est transformé en biogaz.

La formation de biogaz est intense durant les premiers quinze jours et tend à baisser sensiblement après cette période. Le volume du digesteur est calculé en multipliant la quantité de digestat disponible et introduite quotidiennement dans le digesteur et le nombre de jours de rétention. Le résultat donne le volume du digesteur.

### La température

Dans la nature, la formation de biogaz s'étale sur 3 gammes de températures :
a) Les températures psychrophiles: de 10 à 25 °C
b) Les températures mésophiles: de 25 à 38 °C; et
c) Les températures thermophiles: de 49 à 70 °C.

Indépendamment de la température, la quantité de biogaz produite pour une quantité de digestat donnée est la même. Cependant, on retient essentiellement que plus la température est élevée, plus la digestion est rapide, c.-à-d. il faudra moins de temps pour obtenir une même quantité de biogaz. Donc, durant le même temps de rétention, un digesteur de 300 m³ donnera plus de biogaz à la température thermophile qu'a la température mésophile.

### Le contrôle de la quantité, de la qualité, de la température et de la composition du digestat frais

Ces contrôles du digestat introduit quotidiennement réduisent considérablement les problèmes qui pourraient provenir de la digestion thermophile. Ils sont essentiels à la bonne marche de la digestion. Le digestat à introduire doit être tous les jours le même, autant que faire se peut.

### Le rapport carbone / azote

Ce rapport présent dans les produits constitutifs du digestat est déterminé par la recette du digestat. Cette recette comprend principalement des déchets présents sur place et de peu par les déchets étrangers à la ferme ou la petite exploitation agricole.

Selon un premier aspect de l'invention, on utilise un capteur 49 à ultrasons installé dans la cuve de préparation 46. Il sert à mesurer la quantité de matière sèche en suspension dans le digestat présent dans cette cuve. Après une lecture qui détermine le poids de matières sèches présentes, il actionne
a) Un mélangeur 50 à hélice faisant aussi partie intégrante de la cuve qui homogénéise les produits;
b) la pompe 5 qui ajoute des effluents liquides jusqu'à ce que le pourcentage de MS (matière solide) programmé est atteint;
c) une pompe de vidange 52 vidangeant le contenu de la cuve de préparation 46 dans la première cuve 54 du méthaniseur à travers un tuyau zigzaguant 56 dans un échangeur de chaleur 57, aux fins de faire monter la température du digestat en transit à 60 °C.

### Description du principe du capteur à ultrasons

La vitesse ultrasonique d'un liquide dépend de la concentration de ses différents composants. Pour déterminer la vitesse sonique, une pulsation est émise par un émetteur 59 à travers le liquide et le temps précis qu'elle met pour atteindre le receveur 60 est mesuré. La distance entre le transmetteur 59 et le receveur 60 est fixe.

La durée de transmission dans un liquide ayant la concentration type désirée est enregistrée dans le contrôleur de l'appareil. Si la concentration du liquide présent dans la cuve de préparation 46 est supérieure à celle enregistrée, l'appareil déclenche une pompe, par exemple une pompe d'effluent, qui va ajouter du liquide jusqu'à ce que la concentration type soit égalisée. Alors le contrôleur arrête la pompe.

Selon un deuxième aspect de l'invention, la cuve de préparation 46 est montée sur des capteurs de pesage 47, la transformant en une balance ou en un système de pesage qui contrôle le démarrage des pompes et/ou soupapes pour ajouter de liquide (de l'eau fraîche, ou préférablement de l'effluent refoulé par la pompe 5) et/ou des convoyeurs 48 de l'alimentation et leur arrêt automatique une fois le poids programmé de chacun des déchets est atteint.

De cette façon, l'uniformité du digestat à méthaniser est assurée.

Selon un troisième aspect de l'invention, un hachoir en inox 62 est monté sur la cuve de préparation 46. Il sert à la réduction de la longueur de la paille du fumier et des autres déchets d'origines végétales à 3 - 4 mm. Une vis sans fin située, dans le fond de la trémie 63 du hachoir 62, entraîne et force les produits vers un couteau rotatif interchangeable situé à la sortie du hachoir, lequel coupe les produits végétaux à la taille désirée.

Ceci permet de multiplier les surfaces "d'agglutination" des bactéries sur les parties hachées. Pour résultat, on obtient une augmentation de la digestion des matières organiques volatiles contenues dans le digestat et l'augmentation de la production de biogaz tout en réduisant la quantité de ces MSO.

Selon un quatrième aspect de l'invention, on utilise un système de chauffage par diffusion. Les cuves de digestion 3 sont récupérées du matériel roulant des chemins de fer européens et qui était destiné au transport des liquides. Les wagons sur lesquels elles sont montées sont retirés de la circulation à raison de défauts ou d'usures de leurs parties motrices ou roulantes.

Les cuves 3 elles mêmes sont en excellent état. Elles sont traitées pour servir de cuve de méthaniseur.

Ceci réduit le coût total du méthaniseur. L'économie réalisée sert à ajouter et à fournir les équipements tels que le hachoir, les modules de pesage, le capteur à ultrasons, les dispositifs pour l'automatisation, les cuves de chauffage 64, l'isolation etc. Car le système de chauffage des cuves est primordial pour l'efficacité du méthaniseur.

Les systèmes conventionnels présentent les désavantages suivants:
a) Si le système de chauffage est interne et formé de tuyaux en spirale dans la cuve, il sera sujet au tartrage, ce qui le rend inefficace.
b) S'il est externe et formé de tuyaux en spirale soudés aux parois externes des cuves, il y a création de points chauds et de points froids. Ceci diminue son efficacité, et mène à une répartition non-uniforme de la chaleur.

Le méthaniseur prototype préféré est pourvu d'un système de chauffage contournant ces problèmes. Chaque cuve 3 horizontale et cylindrique du méthaniseur est placée et soudée en usine dans une autre cuve extérieure 64 en acier formant ainsi un seul corps. La cuve extérieure 64 est rectangulaire et a la même longueur et largeur que le cylindre de la cuve de méthanisation, sa hauteur est égale au rayon du cylindre plus 24 cm.

La cuve extérieure 64 rectangulaire est remplie d'eau. Cette eau est chauffée par échange avec une partie de l'eau chaude provenant du co-générateur 19. La chaleur de cette eau chaude est transférée vers le haut par le phénomène physique simple de diffusion. Grâce à la grande surface et au grand diamètre de conduits, l'engorgement par tartrage est évité et une distribution uniforme de la chaleur est atteinte.

Le digestat se déplace à partir de la cuve d'entrée 53 par une cuve 3 ver l'autre, en passant par les tuyaux de connexion 11, 12, 13. Il sort de la dernière cuve par un tuyau de sortie 66. La hauteur de ce tuyau 66 dans la paroi de la cuve 3 détermine le niveau 68 du digestat dans les cuves.

Les composants liquides et solides sont séparés par une trémie, et l'effluent est collecté dans un réservoir 70. Par une pompe d'effluent déjà mentionnée plus haut, de l'effluent peut être fourni à la cuve de préparation pour établir la concentration correcte.

Le biogaz développé dans le méthaniseur est collecté par le tuyau de gaz 15 et stocké dans le réservoir de gaz 17. Le réservoir se trouve au-dessus des cuves 3 et consiste essentiellement en une enveloppe flexible.

Afin d'agiter le contenu des cuves 3, un compresseur 9 est connecté avec le réservoir 17 par son entrée, et avec un système de tuyaux d'agitation 72 par sa sortie. Les tuyaux d'agitation 72 sont arrangés au fond des cuves 3 et pourvus d'orifices ou buses 74 par lesquelles le biogaz sous pression 75 échappe en remuant le digestat.

En ce qui concerne le co-générateur, il faut remarquer que son eau de refroidissement provient de l'eau chaude des cuves extérieures 64. Cela permet d'utiliser la chaleur développée par le co-générateur pour chauffer le méthaniseur. De plus, comme déjà mentionné, la masse thermique importante du méthaniseur permet de renoncer à un dispositif de refroidissement d'urgence pour le co-générateur.

Selon un autre aspect important de l'invention, on choisit des matériaux servant à la construction de l'installation, qui ont des faibles coûts énergétiques à la fabrication et à la mise en oeuvre.

La construction du méthaniseur même présente les avantages suivants:
- Pas d'altération du site d'installation;
- pas d'excavation, et
- pas de construction immuable et permanente.

A titre de comparaison, selon la manière conventionnelle de construction d'un méthaniseur, les mesures suivantes sont obligatoires: une cuve en béton, p. ex. de 320 m³, avec un diamètre de 10 m et une profondeur de 4 m, nécessite l'excavation de 500 m³, trois coffrages et 80 m³ de béton armé. Dans le cas où elle est préfabriquée, elle est désagréablement lourd à transporter.

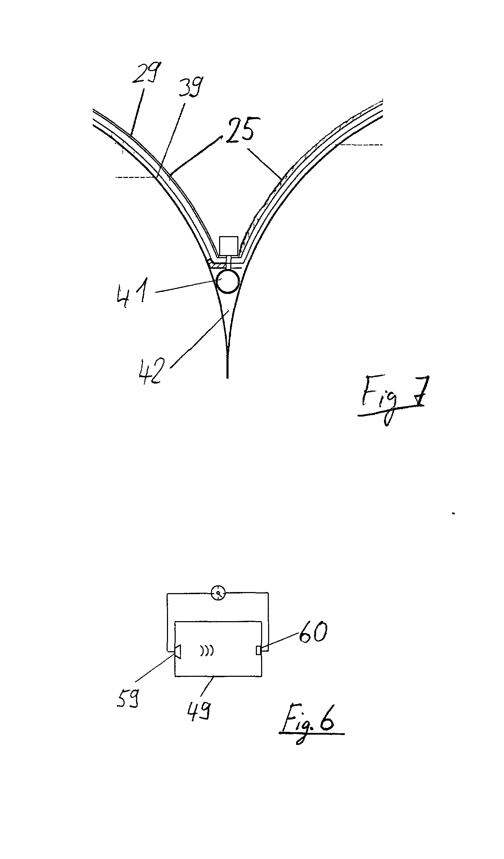

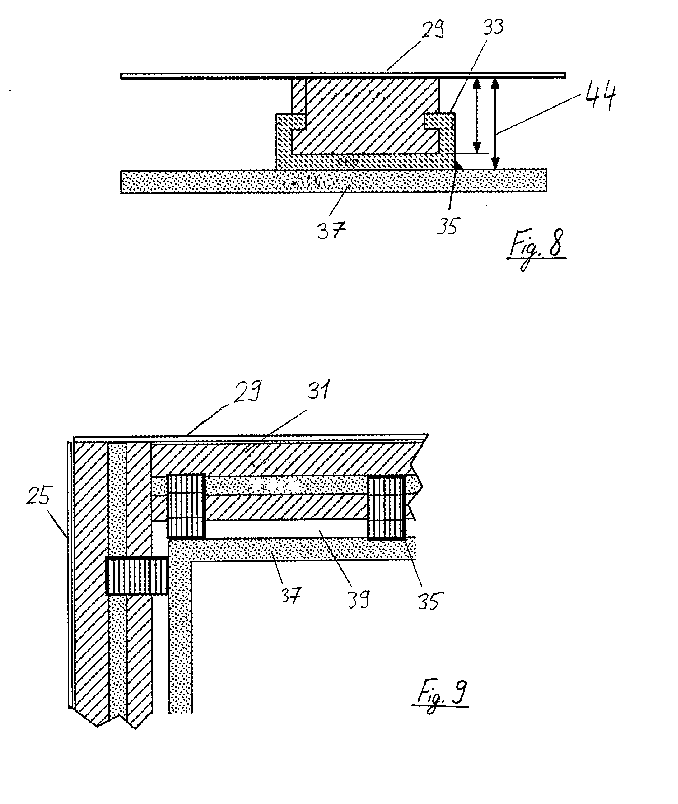

Description des matériaux et de la structure d'un méthaniseur prototype selon l'invention:
- Support: plaques en nid d'abeille 26, pré-assemblées et préfabriquées, de préférence des bouteilles en plastique recyclées, et posées sur une aire nivélée. Des croisillons à leur base leur confèrent un pouvoir portant optimal et une résistance à l'enfoncement. Ils n'entravent pas l'infiltration des eaux pluviales dans le sol, mais retiennent la boue. Elles résistent aux rayons U.V. et au gel et supportent des charges jusqu'à 300 t/m². Elles sont en outre respectueuse de l'environnement.
- Le bardage 25 (Fig. 9) consiste en planches de plastique recyclé pour la protection des parties verticales des cuves externes du méthaniseur et de l'abri du cogénérateur 19 et du tableau de contrôle 27. Les planches sont de type résistant au feu, aux rayons U.V. , au graffitis. Les couleurs sont teintées dans la masse.
- Isolant: On utilise un isolant réflecteur 29 (Fig. 8 et 9). L'isolant est agrafé à des tasseaux écarteurs 31 en bois ignifugé. Les tasseaux 31 sont insérés dans des sections de profilé U 33 en métal soudés (soudage exemplaire 35) aux parois métalliques 37 des cuves, ces tasseaux 31 ont 2 cm de hauteur pour former une lame d'air chaud 39 d'au moins la même épaisseur (à la hauteur des tasseaux s'élève l'épaisseur de la base des profilés U, par exemple 0,5 cm). L'isolant est donc espacé de environ 1 cm des extrémités des profilès U 33. Ainsi, un effet thermos est créé, qui a deux fonctions à la fois :
   1) préserver la température interne du méthaniseur et
   2) utiliser la masse thermique du méthaniseur pour se passer et faire l'économie d'un refroidisseur de secours du co-générateur 19. Le système fonctionne grâce à 3 électrovannes 41 (Fig. 7) placées dans les creux 42 formés entre les parties courbes des cuves internes 3 du méthaniseur.

   L'ouverture des vannes 41 libère l'air chaud emprisonné dans les lames formées par l'isolant et qui sont importantes à ces endroits. Les sondes des thermostats (non dessinées) dont les cuves 3 sont équipées, gèrent le maintien de leur température a 60 °C et celle d'eau de refroidissement du moteur du co-générateur à 71 °C.

L'isolant réflecteur 29 est constitué d'un film de mousse de polyéthylène ignifuge , 25 kg/m² de densité, épaisseur 3 mm, isolé entre 2 lames d'air, d'où une conduction extrêmement faible. Ce film se trouve, similairement à un sandwich, entre deux films de polyéthylène ignifugé et comprend des bulles d'air sec de 150 microns. Le sandwich, à son tour, est en sandwich entre deux films d'aluminium pur (99 % au moins) d'une épaisseur de 30 microns pour refléter le rayonnement à raison de 90% au moins, et aux fins d'obtenir une étanchéité totale à l'air et à l'eau. L'isolant 29 est attaché d'un manière quelconque (agrafé, collé, cloué) aux tasseaux en bois 31 tenus chaque fois par un profilé métallique 35 en U soudé au point 35 aux parois externes 37 des cuves 3 afin de créer une lame d'air 39 étanche vers l'extérieur. La lame d'air a une épaisseur 44 de 2 cm formant un thermos dont le pouvoir isolant est égal à 25 cm d'isolant traditionnel.

Sur la base de la description de l'exemple d'exécution préféré décrit ci-dessus, l'homme de métier peut concevoir des modifications et variantes sans sortir de la portée de protection de l'invention, qui est définie par les revendications.

## Revendications

1. Digesteur-anaérobique, comprenant une cuve de préparation, **caractérisé en ce que** la cuve de préparation (46) est pourvue d'un système de mesure à ultrasons pour mesurer la concentration de la matière solide dans la cuve de préparation et **en ce que** la cuve de préparation est montée sur des capteurs de pesage (47).

2. Digesteur-anaérobique selon la revendication 1, **caractérisé en ce qu'**il comprend un dispositif de diminution de matériau solide, notamment de la paille et d'autres déchets des plantes, préférablement à des particules d'une longueur inférieur à 4 mm, la sortie du dispositif de diminution ménant dans la cuve de préparation (46).

3. Digesteur-anaérobique selon la revendication 1 ou 2, **caractérisé en ce qu'**il comprend au moins une cuve de digestion (3) pour le matériau à digérer, ces cuves de digestions étant chauffées par un système de chauffage par diffusion de chaleur.

4. Digesteur-anaérobique selon la revendication 3, **caractérisé en ce que** les cuves de digestions sont pourvues d'une enveloppe (25) isolante, qui comprend au moins une couche de reflexion de radiation de chaleur et une couche a faible conductivité thermique, et que cette enveloppe est monté d'une distance de la paroi (37) de la cuve pour créer une couche d'air entre l'enveloppe et la cuve.

5. Digesteur-anaérobique selon une des revendications 1 à 4, **caractérisé en ce que** la cuve de préparation (46) est fluidiquement connecté avec la sortie d'effluent des cuves de digestion (3) du digesteur-anaérobique (1) aux fins de pouvoir ajouter de l'effluent au contenu de la cuve de prèparation pour reduire la concentration de matière solide.

6. Digesteur-anaérobique selon une des revendications 1 à 5, **caractérisé en ce que** la cuve de digestion (3) est arrangé dans une cuve extérieure (64) afin de pouvoir contrôler la température de la cuve de digestion (3) par l'ajustage de la température de l'eau dans la cuve extérieure.

7. Digesteur-anaerobique, **caractérisé en ce qu'**il est érigé sur une plate-forme constitué des plaques en nid d'abeilles (26), à savoir des plaques avec des trous polygonaux, de préference hexagonaux, et que les plaques (26) consistent essentiellement en un matériau polymérique.

8. Digesteur-anaerobique selon la revendication 7, **caractérisé en ce que** les plaques (26) sont répandu sur un terrain nivelé pour former la plate-forme.

9. Digesteur-anaérobique selon la revendication 7 ou 8, **caractérisé en ce que** les plaques (26) ont une résistance d'au moins 200 t/m², de préférence d'environ 300 t/m².

10. Digesteur-anaérobique selon une des revendications 7 à 9, **caractérisé en ce que** les trous dans les plaques (26) permettent le passage de l'eau pluviale.

11. Digesteur-anaérobique selon une des revendications 7 à 10, **caractérisé en ce que** les plaques (26) sont pourvues de croisillons pour améliorer leur force portante et la résistance à l'enfoncement.
